# EUROPÄISCHE PATENTSCHRIFT

(11) **EP 0 823 055 B1**
(45) Veröffentlichungstag und Bekanntmachung des Hinweises auf die Patenterteilung: **10.10.2001**
(21) Anmeldenummer: 96903914.8
(22) Anmeldetag: 29.02.1996
(51) Int. Cl.: G01N 33/18, G01N 33/00

(54) **VORRICHTUNG ZUR ERFASSUNG VON IN MEDIEN ENTHALTENEM KOHLENWASSERSTOFF**
DEVICE FOR DETECTING HYDROCARBONS IN MEDIA
DISPOSITIF PERMETTANT DE DETECTER LA PRESENCE D'HYDROCARBURES DANS DES SUBSTANCES

(30) Priorität: 22.04.1995 DE 19514913
(43) Veröffentlichungstag der Anmeldung: 11.02.1998
(73) Patentinhaber: GKSS-FORSCHUNGSZENTRUM GEESTHACHT GMBH, 21502 Geesthacht (DE)
(72) Erfinder: FLÖSER, Götz, D-21037 Hamburg (DE); MASSON, Michel, D-28203 Bremen (DE); WERNECKE, Gerhard, D-30175 Hannover (DE)
(74) Vertreter: Niedmers, Ole, Dipl.-Phys.
(86) Internationale Anmeldenummer: DE9600335
(87) Internationale Veröffentlichungsnummer: WO9634285

(56) Entgegenhaltungen:
- FR-A- 2 682 187
- US-A- 3 719 936
- US-A- 5 110 473

## Beschreibung

Die Erfindung betrifft eine Vorrichtung zur Erfassung von Kohlenwasserstoff in Medien, insbesondere in flüssigen Medien, umfassend ein Sensorelement und ein in einem Abstand zum Element angeordnetes Membranelement, durch das der im Medium enthaltene Kohlenwasserstoff in dem Raum hinter dem Membranelement zur Detektion durch das Element hindurchtritt.

Eine Vorrichtung dieser Art ist bekannt (US-A-3 719 936). Die bekannte Vorrichtung ist dazu bestimmt, auf der Wasseroberfläche zu schwimmen, um beispielsweise auf der Wasseroberfläche schwimmendes Öl, das aufgrund von Schiffsunfällen oder aber auch beabsichtigterweise durch das Bilgenwasser oder aufgrund sonstiger Ereignisse auf die Wasseroberfläche gelangte, zu detektieren. Dazu weist die Vorrichtung ein wenigstens teilweise schwimmendes Gehäuse auf, das am Boden mit einem Ballastmittel versehen ist, so daß die Vorrichtung im wesentlichen vertikal aufrecht schwimmt. Zur Oberfläche des Wassers hin gerichtet ist in der Vorrichtung eine Membraneinrichtung vorgesehen, durch die auf dem Wasser befindliches Öl hindurchtritt und in eine trichterförmige Kammer eintritt. Die Kammer muß vollständig mit Öl gefüllt sein, um den Sensor innerhalb der Kammer berühren zu können.

Aus der FR-A-2 682 187 ist eine Vorrichtung und ein Verfahren zum Bestimmen strippbarer Substanzen aus Flüssigkeiten, wie insbesondere halogenierter Kohlenwasserstoff in Prozeß- oder Abwässern, bekannt. Mittels der bekannten Vorrichtung und dem bekannten Verfahren soll eine kontinuierliche Bestimmung der Substanzen aus fließenden Flüssigkeiten in Echtzeit möglich sein, so daß beispielsweise in der chemischen Industrie Regelungsvorgänge hinsichtlich der Konzentration von bestimmten Substanzen in einer Flüssigkeit vorgenommen werden können sollen. Dazu werden die Flüssigkeiten an einer füssigkeitsundurchlässigen aber gasdurchlässigen Membran vorbeigeführt, so daß die sprippbaren Substanzen in der Flüssigkeit in ihrer Gasphase durch die Membran hindurchtreten und in eine Meßzelle geleitet werden. Als Meßzelle wird dort beispielsweise eine Elektronenstoßionenquelle eines Quadropolmassenspektrometers angeführt, wobei das Quadropolmassenspektrometer einen Sekundärelektronenvervielfacher aufweist.

Für die Erdöl- und Erdgasexploration im Meer und für die kontinuierliche Überwachung bestehender Explorations- und Fördereinrichtungen von Erdöl und Erdgas, aber auch für den Nachweis von Kohlenwasserstoffen in Sedimenten von Flüssen und Meeren ist ein auf die Anmelderin selbst zurückgehendes Nachweissystem für Kohlenwasserstoffe unter der Bezeichnung MEDUSA (Methane Detection for Under-Sea Application) bekannt, das direkt vor Ort von Schiffen und Plattformen aus eingesetzt werden kann. Mittels des vorgenannten Nachweissystems MEDUSA können extrem niedrige Methanmengen pro Wasservolumen nachgewiesen werden, wobei das bekannte System zum Nachweis des Kohlenwasserstoffs eine Lasereinrichtung benutzt.

Das bekannte System ist aufgrund seines Aufbaus, seiner ursprünglichen Bestimmung und seiner Konzeption zum massenweisen Einsatz weniger geeignet.

Es besteht nämlich das Bedürfnis, beispielsweise Fördereinrichtungen von Erdöl und Erdgas im Meer fortlaufend zuverlässig auf aus dem Meeresboden aufsteigende Kohlenwasserstoffe bzw. von den Fördereinrichtungen selbst abgegebene Kohlenwasserstoffe hin zu überwachen, um bei detektierten Kohlenwasserstoffen sofort Gegenmaßnahmen ergreifen zu können, indem beispielsweise erkannte Lecks in Förder- oder Bohrleitungen sofort abgedichtet werden können und dadurch ökologische Beeinträchtigungen und wirtschaftliche Verluste vermieden werden können. Derartige unmittelbare Überwachungen von Fördereinrichtungen, Förderleitungen und Bohrlöchern bzw. Bohrleitungen wurden bisher beispielsweise mit Videokameras durchgeführt oder es wurde der Förderstrom durch eine Förderleitung gemessen und Veränderungen, die auf Lecks hindeuten, registriert. Wenn Lecks jedoch klein sind, sind sie durch derartige Überwachungs- bzw. Meßmethoden nur unzureichend, wenn überhaupt, zu bemerken, so daß sich diese Methode als ungenau, unzuverlässig und auch kostspielig erwiesen hat, zumal Lecks dieser Art zuverlässig dann erst durch Überwachung der Meeresoberfläche aus der Luft erkannt werden können, was nur mit großem Kostenaufwand möglich ist und in vielen Fällen schon irreparable Beeinträchtigungen des Wassers und/oder der Umgebung eingetreten sind.

Es ist Aufgabe der vorliegenden Erfindung, eine Vorrichtung der eingangs genannten Art zu schaffen, die sich für den massenweisen Einsatz beispielsweise bei Fördereinrichtungen, Förderleitungen, Bohrlöchern oder dergleichen für Erdöl- und Erdgasförderung im Meer eignet und auch zur Erfassung von Kohlenwasserstoffen in Sedimenten der Flüsse und der Meere, die zuverlässig hochgenaue Meßergebnisse liefert und die einfach im Aufbau und damit kostengünstig herstellbar ist.

Gelöst wird die Aufgabe gemäß der Erfindung dadurch, daß das Sensorelement ein kohlenwasserstoffsensitives Element ist, wobei das Membranelement derart beschaffen ist, daß nur Kohlenwasserstoffgas in den als Gasraum ausgebildeten Raum gelangen kann.

Der Raum zwischen Membranelement und dem kohlenwasserstoffsensitiven Element ist der eigentliche Meßraum, der klein gehalten wird, um die Ansprechzeit des kohlenwasserstoffsensitiven Elements, das regelmäßig ein Halbleitersensorelement ist, zu minimieren. Der Vorteil der erfindungsgemäßen Vorrichtung besteht darin, daß durch das Membranelement lediglich Kohlenwasserstoffe hindurchdringen, hingegen das Medium, in dem die Kohlenwasserstoffe in gelöster oder ungelöster Form enthalten sind, durch das Membranelement, das für das Medium als Barriere wirkt, vom Eindringen in den Gasraum ferngehalten wird. Ein weiterer Vorteil ist, daß aufgrund des verhältnismäßig einfachen Aufbaus der Vorrichtung diese sich, wie angestrebt, zum massenweisen Einsatz beispielsweise zur ständigen Überwachung von Erdöl- und Erdgasfördereinrichtungen im Meer einschließlich der Förderleitungen eignet, so daß diese zu einem Netzwerk verbunden an einer zentralen Stelle fortwährend Auskunft über die Konzentration von im Wasser gelösten Kohlenwasserstoffen liefert und bei der Erfassung erhöhter Konzentrationen Gegenmaßnahmen zur Bekämpfung von Lecks, die sich somit leicht lokalisieren lassen, eingeleitet werden können.

Schließlich bietet die erfindungsgemäße Lösung den Vorteil, daß damit auch Kohlenwasserstoffquellen am Meeresboden für geologische und/oder biologische Zwecke erfaßt werden können, so daß die bisherigen sogenannten "Trail-and-Error Methoden", d.h. in der Umgebung einer vermuteten Kohlenwasserstoffquelle solange Proben zu nehmen, bis man die richtige Stelle durch Zufall gefunden hat, und bei den Ausbeuten von lediglich bisher 10% erwartet werden, entfallen und auch für diese Zwecke eine sichere Methode zur Verfügung steht, mit hoher Wahrscheinlichkeit und geringem Aufwand Kohlenwasserstoffquellen in großer Tiefe schnell entdecken zu können.

Gemäß einer vorteilhaften Ausführungsform umfaßt die Vorrichtung ein druckdichtes, im Bereich des Membranelementes zum Medium offenes Gehäuse, in dem wenigstens das Membranelement und das kohlenwasserstoffsensitive Element angeordnet sind. Das druckdichte Gehäuse ist derart ausgelegt, daß es dem der Einsatztiefe zu erwartenden Druck ohne weiteres standhält, d.h. es sind prinzipiell Gehäuse, die unterschiedlichen Drücken standzuhalten vermögen, denkbar, und zwar je nach beabsichtigter Einsatztiefe der Vorrichtung im Meer.

Bei einer vorteilhaften Ausgestaltung der Vorrichtung ist das flächig ausgebildete Membranelement auf einer gasdurchlässigen, zwischen Membranelement und kohlenwasserstoffsensitivem Element angeordneten Platte angeordnet, wodurch erreicht wird, daß durch die Anlage des Membranelements an der gasdurchlässigen Platte eine hohe mechanische Stabilität des Membranelementes auf einfache Weise gewährleistet ist, die den hohen zu erwartenden Drücken beim Einsatz der Vorrichtung standhält.

Obwohl die gasdurchlässige Platte prinzipiell aus jedem beliebigen geeigneten Werkstoff hergestellt werden kann, der die Gasdurchlässigkeit bei hoher mechanischer Stabilität gewährleistet, d.h. Platten, die ausreichend mechanisch stabil bei ausreichender Porosität zum Durchlaß des durch die Membran permeierenden Kohlenwasserstoffs sind, ist es insbesondere vorteilhaft, die Platte aus gesintertem Metall aufzubauen, wobei gesintertes Metall einerseits mechanisch stabil ist und andererseits eine ausreichende Porosität besitzt, um das durch das Membranelement hindurchtretende Kohlenwasserstoffgas in den Gasraum behinderungsfrei eintreten zu lassen.

Um das Membranelement bei Beschädigungen im Zuge von Wartungsmaßnahmen oder auch zum Austausch gegen ein anderes Membranelement bei anderen Anwendungszwecken der Vorrichtung schnell zugänglich zu machen, ist das Membranelement zusammen mit der Platte mittels einer Überwurfmutter über ein umlaufendes Dichtelement gegenüber dem Gehäuse lösbar dichtend verbindbar. Dadurch kann schnell das Membranelement entfernt, überprüft oder gegen ein neues ausgetauscht werden.

Das kohlenwasserstoffsensitive Element, das grundsätzlich einen beliebigen geeigneten Aufbau haben kann und beispielsweise ein sogenannter "Figaro-Gassensor" der Figaro Engineering Inc. sein kann, ist vorzugsweise lösbar steckbar auf einem Sockelelement aufnehmbar, so daß für das kohlenwasserstoffsensitive Element genau das gleiche wie bei der vorangehend geschilderten lösbaren Befestigungsmöglichkeit des Membranelementes gilt, daß nämlich das kohlenwasserstoffsensitive Element aufgrund seiner Steckbarkeit für Wartungs- oder Austauschzwecke entfernt und wiederum leicht darauf angeordnet werden kann.

Grundsätzlich kann die Vorrichtung vorzugsweise über nach außen führende Datenverbindungsleitungen und/oder nach außen führende Spannungsversorgungsleitungen extern beispielsweise von einem Schiff, einer Beobachtungsstation, einer Bohrplattform oder dergleichen betrieben werden, so daß in der eigentlichen Vorrichtung lediglich das kohlenwasserstoffsensitive Element angeordnet ist. Für bestimmte Anwendungszwecke kann es jedoch vorteilhaft sein, zumindest im Innenraum des Gehäuses eine Spannungsquelle und/oder eine Elektronikeinrichtung zum Betrieb des kohlenwasserstoffsenitiven Elements vorzusehen, so daß ggf. lediglich eine externe Grundspannungsversorgung von außen über die Spannungsversorgungsleistung der Vorrichtung zugeführt werden muß und lediglich die Datenleitungen vom kohlenwasserstoffsensitiven Element nach außen geführt werden müssen. Beide Ausführungsformen der vorbeschriebenen Art sind bei der erfindungsgemäßen Vorrichtung denkbar.

Die Erfindung wird nun unter Bezugnahme auf die einzige schematische Zeichnung anhand eines Ausführungsbeispieles eingehend beschrieben. Diese zeigt:
Im Schnitt in annähernd maßstabgerechter Größe eine Ausführungsform der Vorrichtung zur Erfassung von in Medien enthaltenem Kohlenwasserstoff.

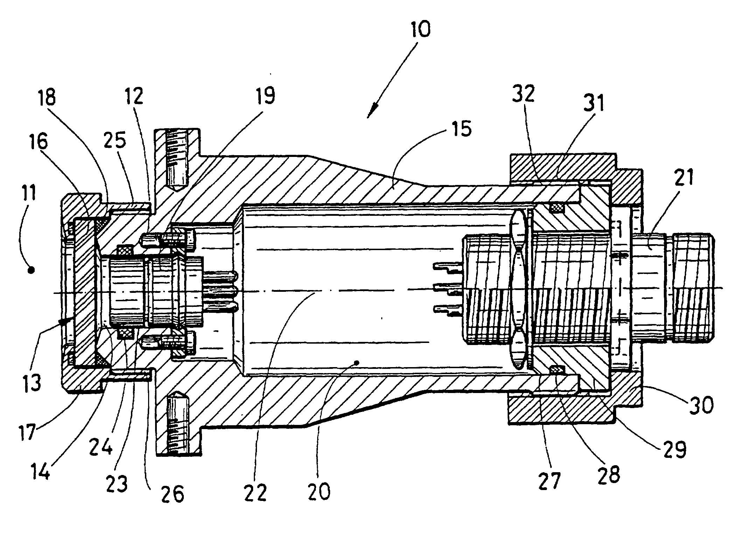

Die Vorrichtung 10 besteht im wesentlichen aus einem Gehäuse 15, das rotationssymmetrisch zu einer Achse 22 ist. Das Gehäuse 15 weist einen Innenraum 14 auf, der zur Aufnahme hier nicht dargestellter Elemente wie einer einer Elektronikeinrichtung zum Betrieb der Vorrichtung und dergleichen dient, ggf. auch einer Spannungsquelle. Ein Ende der regelmäßig als Drehkörper ausgebildeten Vorrichtung 10, bei Betrachtung der einzigen Figur links, ist mit einer Öffnung 23 versehen, in der über ein umlaufendes Dichtelement 24 ein kohlenwasserstoffsensitives Element 12, im folgenden Sensorelement genannt, dichtend angeordnet ist. Das Sensorelement 12 sitzt auf einem Sockelelement 19, das im Bereich des an den Innenraum 14 angrenzenden Endes der Öffnung 23 mittels geeigneter Befestigungseinrichtungen mit dem Gehäuse 15 verbunden ist.

Das Sensorelement 12 ist über ein umlaufendes Dichtelement 24, beispielsweise in Form eines O-Ringes, gegenüber dem Innenraum 14 abgedichtet.

Mittels einer Überwurfmutter 17, die über ein Innengewinde 25 mit einem am Gehäuse 15 an entsprechender Stelle ausgebildeten Außengewinde 26 verbindbar ist, ist ein auf einer kohlenwasserstoffdurchlässigen Platte 16 angeordnetes flächiges Membranelement 13 über ein umlaufendes Dichtelement 18, beispielsweise in Form eines O-Ringes, dichtend verbindbar, so daß zwischen der Platte 16 und dem Sensorelement 12 ein Raum 14, der eigentliche Gasraum, gebildet wird. Mittels der Überwurfmutter 17 kann somit das Membranelement 13 für Wartungs-, Austausch- oder sonstige Zwecke leicht entfernt und an dieser Stelle wiederum leicht positioniert werden. Das Membranelement 13 besteht beispielsweise aus einer beschichteten integralasymmetrischen Membran, wie sie in der DE-PS 37 16 916 der jetzigen Patentanmelderin beschrieben worden ist. Grundsätzlich können aber auch beliebige andere geeignete Polymermembranen für die erfindungsgemäße Vorrichtung 10 eingesetzt werden.

Die der Öffnung 23 gegenüberliegende andere Öffnung 27 der Vorrichtung 10 ist mittels eines Verschlußelementes 29 und ein damit zusammenwirkendes, umlaufendes Dichtelement, beispielsweise in Form eines O-Ringes 28, verschlossen, wobei das Verschlußelement 29 wiederum über eine Überwurfmutter 30 mittels daran ausgebildetem Innengewinde 31 und am Gehäuse 15 an geeigneter Stelle ausgebildetem Außengewinde 32 mit dem Gehäuse 15 dichtend verbindbar ist. Im Verschlußelement 29 ist eine Steckereinrichtung 21 dichtend angeordnet. Die Steckereinrichtung 21 dient dem Anschluß beispielsweise einer nach außen zu einer Bohrplattform, einem Schiff oder dergleichen führenden, hier nicht dargestellten Datenverbindungsleitung und/oder einer entsprechend nach außen führenden Spannungsversorgungsleitung. Mittels der Überwurfmutter 30 ist ebenfalls auf einfache Weise ein Zugang zum Gehäuseinnenraum 20 möglich, d.h. zur Reparatur, zu Wartungs- und Austauschzwecken und dergleichen.

Die Vorrichtung 10 arbeitet folgendermaßen.

Die im Medium 11 an geeigneter Stelle und geeigneter Tiefe entweder stationär oder bewegbar angeordnete Vorrichtung 10 erfaßt im Medium 11, beispielsweise Wasser, vorhandenen Kohlenwasserstoff, indem der Kohlenwasserstoff durch das Membranelement 13 hindurchpermeiert und durch die poröse Platte 16, beispielsweise aus gesintertem Metall, in den Raum 14, den eigentlichen Gasraum, gelangt. Der Raum 14 bzw. der Gasraum wird gebildet durch die vordere, bezogen auf die einzige Figur, linke Fläche des Sensorelements 12 und durch die, bezogen auf die einzige Figur, rechte Begrenzungsfläche der Platte 16. Das Volumen des Raumes 14 wird klein gehalten, um die Ansprechzeit des kohlenwasserstoffsensitiven Elements 12 zu minimieren. Die Ansprechzeit des Sensorelements 12 liegt im Bereich von 1 bis 15 Minuten.

Erkennt das Sensorelement 12 im Gasraum 14 befindlichen Kohlenwasserstoff, werden von diesem geeignete Signale generiert, die entweder von der im Gehäuseinnenraum 20 angeordneten Elektronikeinrichtung aufbereitet über die Steckereinrichtung 21 über hier nicht dargestellte Datenverbindungsleitungen an die Förderplattform, ein Schiff oder dergleichen gegeben werden oder aber ggf. ohne vorherige Signalaufbereitung direkt über die Steckereinrichtung 21 an die vorbenannten Stellen.

### Bezugszeichenliste

- 10: Vorrichtung
- 11: Medium
- 12: kohlenwasserstoffsensitives Element (Sensorelement)
- 13: Membranelement
- 14: Raum
- 15: Gehäuse
- 16: Platte
- 17: Überwurfmutter
- 18: Dichtelement
- 19: Sockelelement
- 20: Gehäuseinnenraum
- 21: Steckereinrichtung
- 22: Achse
- 23: Öffnung
- 24: Dichtelement
- 25: Innengewinde
- 26: Außengewinde
- 27: Öffnung
- 28: Dichtelement
- 29: Verschlußelement
- 30: Überwurfmutter
- 31: Innengewinde
- 32: Außengewinde

## Patentansprüche

1. Vorrichtung zur Erfassung von Kohlenwasserstoff in Medien, insbesondere in flüssigen Medien, umfassend ein Sensorelement (12) und ein in einem Abstand zum Element (12) angeordnetes Membranelement (13), durch das der im Medium (11) enthaltene Kohlenwasserstoff in den Raum (14) hinter dem Membranelement (13) zur Detektion durch das Element (12) hineintritt, **dadurch gekennzeichnet, daß** das Sensorelement (12) ein kohlenwasserstoffsensitives Element (12) ist, wobei das Membranelement (13) derart beschaffen ist, daß nur Kohlenwasserstoffgas in den als Gasraum ausgebildeten Raum (14) gelangen kann.

2. Vorrichtung nach Anspruch 1, **dadurch gekennzeichnet, daß** diese ein druckdichtes, im Bereich des Membranelementes (13) zum Medium (11) offenes Gehäuse (15) umfaßt, in dem wenigstens das Membranelement (13) und das kohlenwasserstoffsensintive Element (12) angeordnet sind.

3. Vorrichtung nach einem oder beiden der Ansprüche 1 oder 2, **dadurch gekennzeichnet, daß** das flächig ausgebildete Membranelement (13) auf einer gasdurchlässigen, zwischen Membranelement (13) und kohlenstoffsensensitivem Element (12) angeordneten Platte (16) angeordnet ist.

4. Vorrichtung nach Anspruch 3, **dadurch gekennzeichnet, daß** die Platte (16) aus gesintertem Metall besteht.

5. Vorrichtung nach einem oder beiden der Ansprüche 3 oder 4, **dadurch gekennzeichnet, daß** das Membranelement (13) zusammen mit der Platte (16) mittels einer Überwurfmutter (17) über ein umlaufendes Dichtelement (18) gegenüber dem Gehäuse (15) lösbar dichtend verbindbar ist.

6. Vorrichtung nach einem oder mehreren der Ansprüchel bis 5, **dadurch gekennzeichnet, daß** das kohlenwasserstoffsensitive Element (12) lösbar steckbar auf einem Sockelelement (19) aufnehmbar ist.

7. Vorrichtung nach einem oder mehreren der Ansprüche 1 bis 6, **dadurch gekennzeichnet, daß** im Innenraum (20) des Gehäuses (15) eine Spannungsquelle und/oder eine Elektronikeinrichtung zum Betrieb des kohlenwasserstoffsensitiven Elements (12) aufnehmbar ist.

8. Vorrichtung nach einem oder mehreren der Ansprüche 1 bis 7, **dadurch gekennzeichnet, daß** eine nach außen führende Datenverbindungsleitung und/oder eine nach außen führende Spannungsversorgungsleitung an eine vorrichtungsseitige Steckereinrichtung (21) anschließbar ist bzw. sind.

## Claims

1. Device for detecting hydrocarbon in media, especially in liquid media, comprising a sensor element (12) and, arranged at a distance from the element (12), a membrane element (13) through which the hydrocarbon contained in the medium (11) enters the space (14) behind the membrane element (13) for detection by the element (12), **characterized in that** the sensor element (12) is a hydrocarbon-sensitive element (12), whereby the membrane element (13) is constructed in such a way that only hydrocarbon gas can enter the space (14) which is designed in the form of a gas space.

2. Device according to Claim 1, **characterized in that** it comprises a pressure-tight housing (15), open towards the medium (11) in the region of the membrane element (13), in which at least the membrane element (13) and the hydrocarbon-sensitive element (12) are arranged.

3. Device according to one or both of the Claims 1 or 2, **characterized in that** the membrane element (13) designed as a two-dimensional flat surface is positioned on a gas-permeable plate (16) arranged between the membrane element (13) and the carbon-sensitive element (12).

4. Device according to Claim 3, **characterized in that** the plate (16) consists of sintered metal.

5. Device according to one or both of the Claims 3 or 4, **characterized in that** the membrane element (13) together with the plate (16) is detachably attachable so as to seal relative to the housing (15) via a peripheral sealing element (18) by means of a union nut (17).

6. Device according to one or more ofthe Claims 1 to 5, **characterized in that** the hydrocarbon-sensitive element (12) can be accommodated detachably by being plugged onto a base element (19).

7. Device according to one or more of the Claims 1 to 6, **characterized in that** a voltage source and/or an electronics equipment to operate the hydrocarbon-sensitive element (12) can be accommodated in the internal cavity (20) of the housing (15).

8. Device according to one or more of the Claims 1 to 7, **characterized in that** a data connection cable leading to the exterior and/or a voltage supply cable leading to the exterior is/are attachable to a plug equipment (21) on the device side.

## Revendications

1. Dispositif pour la détection d'hydrocarbures dans des milieux, en particulier dans des milieux liquides, comprenant un élément capteur (12) et, disposé à une certaine distance de l'élément (12), un élément membrane (13) à travers lequel l'hydrocarbure contenu dans le milieu (11) pénètre dans l'espace (14) à l'arrière de l'élément membrane (13) pour la détection par l'élément (12), **caractérisé en ce que** l'élément capteur (12) est un élément sensible aux hydrocarbures (12), l'élément membrane (13) étant réalisé de façon à ce que seuls des hydrocarbures à l'état gazeux puissent parvenir dans l'espace (14) sous forme d'espace pour gaz.

2. Dispositif selon la revendication 1, **caractérisé en ce qu'**il comprend un boîtier (15) étanche à la pression, ouvert sur le milieu (11) dans la zone de l'élément membrane (13) dans lequel sont disposés au moins l'élément membrane (13) et l'élément sensible aux hydrocarbures (12).

3. Dispositif selon l'une des revendications 1 et 2 ou les deux, **caractérisé en ce que** l'élément membrane (13) à structure plane est disposé sur une plaque perméable aux gaz (16) disposée entre l'élément membrane (13) et l'élément sensible aux hydrocarbures (12).

4. Dispositif selon la revendication 3, **caractérisé en ce que** la plaque (16) se compose de métal fritté.

5. Dispositif selon l'une des revendications 3 et 4 ou les deux, **caractérisé en ce que** l'élément membrane (13) conjointement avec la plaque (16) peut être relié de manière amovible et étanche vis-à-vis du boîtier (15) par l'intermédiaire d'un élément d'étanchéité tournant (18) au moyen d'un écrou d'accouplement (17).

6. Dispositif selon l'une ou plusieurs des revendications 1 à 5, **caractérisé en ce que** l'élément sensible aux hydrocarbures (12) peut être reçu de manière amovible par enfichage sur un élément de socle (19).

7. Dispositif selon l'une ou plusieurs des revendications 1 à 6, **caractérisé en ce qu'**une source de tension et/ou un dispositif électronique peut être reçu(e) dans l'espace intérieur (20) du boîtier (15) pour faire fonctionner l'élément sensible aux hydrocarbures (12).

8. Dispositif selon l'une ou plusieurs des revendications 1 à 7, **caractérisé en ce qu'**un câble de liaison de données conduisant vers l'extérieur et/ou un câble d'alimentation en tension conduisant vers l'extérieur peut ou peuvent être relié(s) à un dispositif de connexion (21) côté dispositif.
